# NEW EUROPEAN PATENT SPECIFICATION

(11) **EP 1 317 294 B2**
(45) Date of publication and mention of the opposition decision: **02.09.2009**
(45) Mention of the grant of the patent: 16.03.2005
(21) Application number: 01961846.1
(22) Date of filing: 02.08.2001
(51) Int. Cl.: A61L 24/04, C09J 4/00

(54) **ABSORBABLE ADHESIVE COMPOSITIONS**
RESORBIERBARE KLEBSTOFFZUSAMMENSETZUNGEN
COMPOSITIONS ADHESIVES ABSORBABLES

(30) Priority: 02.08.2000 US 630437
(43) Date of publication of application: 11.06.2003
(73) Proprietor: Closure Medical Corporation, Raleigh, North Carolina 27616 (US)
(72) Inventor: JONN, Jerry, Y., Raleigh, NC 27614 (US); BOBO, John, Raleigh, NC 27615 (US); QUINTERO, Julian, Raleigh, NC 27616 (US); MOSELEY, Jon, P., Arlington, TN 38002 (US); BURNS, Dennis, D., Raleign, NC 27615 (US)
(74) Representative: James, Anthony Christopher W.P.
(86) International application number: PCT/US2001/024128
(87) International publication number: WO 2002/009785

(56) References cited:
- EP-A- 0 374 252
- EP-A- 0 965 623
- WO-A-01/32319
- WO-A-97/31598
- DE-A- 1 939 625
- DE-A1- 2 617 525
- JAFFE H ET AL: "Synthesis and bioevaluation of alkyl 2-cyanoacryloyl glycolates as potential soft tissue adhesives" JOURNAL OF BIOMEDICAL MATERIALS RESEARCH, vol. 20, no. 2, 1986, pages 205-212, XP001058226
- 3RD EDITION: 'Principles of Polymerization', 1991, JOHN WILEY & SONS, INC., NEW YORK, ISBN 0-471-61020-8 article ODIAN, GEORGE, pages 496 - 497

## Description

### BACKGROUND OF THE INVENTION

### 1. Field of the Invention

The invention relates to biocompatible adhesive compositions, and to their use, and to kits for the preparation of the compositions.

### 2. State of the Art

Monomer and polymer adhesives are used in both industrial (including household) and medical applications. Included among these adhesives are the 1,1-disubstituted ethylene monomers and polymers, such as the α-cyanoacrylates. Since the discovery of the adhesive properties of such monomers and polymers, they have found wide use due to the speed with which they cure, the strength of the resulting bond formed, and their relative ease of use. These characteristics have made α-cyanoacrylate adhesives the primary choice for numerous applications such as bonding plastics, rubbers, glass, metals, wood, and, more recently, biological tissues.

Medical applications of 1,1-disubstituted ethylene adhesive compositions include use as an alternate or an adjunct to surgical sutures and staples in wound closure as well as for covering and protecting surface wounds such as lacerations, abrasions, burns, stomatitis, sores, and other surface wounds. When an adhesive of this type is applied, it is usually applied in its monomeric form, and the resultant polymerization gives rise to the desired adhesive bond.

For example, polymerizable 1,1-disubstituted ethylene monomers, and adhesive compositions comprising such monomers, are disclosed in U.S. Patent No. 5,328,687 to Leung et al. Suitable methods for applying such compositions to substrates, and particularly in medical applications, are described in, for example, U.S. Patents Nos. 5,928,611; 5,582,834; 5,575,997; and 5,624,669, all to Leung et al.

Some monomeric α-cyanoacrylates are extremely reactive, polymerizing rapidly in the presence of even minute amounts of an initiator, including moisture present in the air or on moist surfaces such as animal tissue. Monomers of α-cyanoacrylates are anionically polymerizable or free radical polymerizable, or polymerizable by zwitterions or ion pairs to form polymers. Once polymerization has been initiated, the cure rate can be very rapid to very slow, depending on the choice of monomer.

However, not all cyanoacrylates polymerize at the same rate; and therefore, various initiators have been added to cyanoacrylates to induce polymerization. For example, each of U.S. Patents Nos. 5,928,611 to Leung; 5,902,443 to Kanakubo et al.; 4,460,759 to Robins; 4,378,213 to Severy; 5,066,743 and 4,979,993 to Okamoto et al.; 5,262,200 to Puder et al.; 4,012,402 and 3,903,055 to Buck; and 3,940,362 to Overhults discloses cyanoacrylate monomers polymerized by the addition of various initiators. The compositions are however directed to catalyzing reactions which only require minor stimulation or initiation to occur. U.S. Patent No. 5,079,098 to Liu also addresses the addition of initiators to cyanoacrylates, but only for the purpose of promoting increased bonding.

U.S. Patent No. 5,928,611 to Leung broadly discloses 1,1-disubstituted ethylene monomers having a large number of possible substituent groups. The disclosure focuses on alpha cyanoacrylates, with alternative representation of ester cyanoacrylates having an organic radical substituent. However, the disclosure does not specify particular properties, such as absorbability, possessed by particular cyanoacrylates. The disclosure does not indicate which initiators work well with which cyanoacrylates. Not all cyanoacrylates work well with all initiators. The disclosure also does not disclose absorption rates, or the effect of the selection of initiators on the properties possessed by cyanoacrylates or polymerization products thereof.

U.S. Patent No. 3,995,641 to Kronenthal et al. discloses absorbable carbalkoxyalkyl 2-cyanoacrylates. The disclosure does not discuss the use of initiators, but rather indicates that blood and other body fluids polymerize the monomers. The disclosure also does not address the effect of the selection of initiators on the properties possessed by cyanoacrylates or polymerization products thereof.

Absorbable adhesives have additional benefits over non-absorbable adhesives under some circumstances, particularly for some medical applications. However, some absorbable cyanoacrylate adhesive compositions have particularly slow reaction kinetics which reduce their practical value as surgical adhesives. Therefore, there is still a need for an adhesive composition that combines absorbability and a rapid cure rate sufficient for medical applications.

In addition, of the various monomer compositions that can be used for medical and surgical purposes, degradation of the resultant formed polymer film is often a concern. Previously, it has been difficult to adjust the degradation rates and other chemical properties of the polymer film. Therefore, there is still a need for an adhesive composition that allows for the tailoring of the degradation rate and other properties, to fit a particular desired use.

Some effort has been made in the field to produce absorbable cyanoacrylate polymer materials. For example, U.S. Patent No. 6,224,622 discloses bioabsorbable cyanoacrylate-based tissue adhesives containing bioabsorbable copolymers. The copolymers are preferably derived from ε-caprolactone, lactide and glycolide monomers or from butyl 2-cyanoacrylate, glycolide, lactide, ε-caprolactone monomers. The adhesives are described to have increased biodegradability, increased viscosity and increased flexibility. The adhesives are useful for wound and incision closure, and for medical devices, including implants. The adhesive can include a cyanoacrylate monomer or a blend of cyanoacrylate monomers, where the cyanoacrylate monomer or monomers are selected from the group consisting of alkyl 2-cyanoacrylate, alkenyl 2-cyanoacrylate, alkoxyalkyl 2-cyanoacrylate, and carbalkoxyalkyl 2-cyanoacrylate, and wherein the alkyl group of said cyanoacrylate monomers has 1 to 16 carbon atoms.

WO 00/72761 also discloses blends of absorbable materials including glycolide, lactide, caprolactone, dioxanone, trimethylene carbonate, alkylene glycols, and esteramides with cyanoacrylate. The blend of materials is described as being absorbable and providing flexibility and adhesive properties, while maintaining acceptable viscosity and curing time.

EP-A-096563 discloses adhesive composition comprising at least one monomer selected from a group of alkylester cyanoacrylate and the use of benzalkonium chloride as both a medicament and polymerization inhibitor, such composition also comprising two vapor phases with one vapor and one liquid phase anionic stabilizer. The monomeric composition, that polymerize only after being applied to a wound, can be sterilized without unacceptable levels of polymerization occurring and provides a sterile monomer containing adhesive composition that has an improved shelf-life and a high monomer, to polymer ration.

EP-A-0374252 discloses also the use of alkyl cyanoacrylates as monomer adhesive compositions, such compositions being haemostatic and may be injected into the site of bleeding by means of endoscopic needle before rapidly polymerizing to seal the lesion permanently.

WO 97 31598A discloses biocompatible monomer adhesive composition, comprising alkyl ester cyanoacrylates and at least two different monomers and an acid stabilizing agent and optionally a formaldehyde scavenger to reduce toxicity. The composition is used for joining tissues and as sealants for retarding or preventing bleeding or for covering open wounds.

### SUMMARY OF THE INVENTION

Benefits of biocompatible adhesives of the invention include ease and rapidity of application, which may be accompanied by inhibition of microbial growth, and lower cost than sutures or staples.

The present invention provides a biocompatible adhesive composition as defined in claim 1. The composition can be used in a method of treating living tissue, comprising applying to living tissue the bi.ocompatible adhesive composition, and allowing the composition to polymerize. The combination of a faster absorbing monomer species and non-absorbable (or less absorbable or slower absorbing) monomer species allows for adjustment and tailoring of the degradation rate of the resultant formed polymer.

For the purposes of this invention, the terms "absorbable" or "absorbable adhesive" or variations thereof mean the ability of a tissue-compatible material to degrade or biodegrade at some time after implantation into products that are eliminated from the body or metabolized therein. Thus, as used herein, absorbability means that the polymerized adhesive is capable of being absorbed, either fully or partially, by tissue after application of the adhesive. Likewise, the terms "non-absorbable" or "non-absorbable adhesive" or variations thereof mean completely or substantially incapable of being absorbed, either fully or partially, by tissue after application of the adhesive. Furthermore, relative terms such as "faster absorbing" and "slower absorbing" are used relative to two monomer species to indicate that a polymer produced from one monomer species is absorbed faster (or slower) than a polymer formed from the other monomer species.

For the purposes of this invention, the term "substantially absorbed" means at least 90% absorbed within about three years. Likewise, the term "substantially non-absorbed" means at most 20% absorbed within about three years.

For the purposes of this invention, the term "biocompatible" refers to a material being suited for and meeting the requirements of a medical device, used for either long or short term implants or for non-implantable applications, such that when implanted or applied in an intended location, the material serves the intended function for the required amount of time without causing an unacceptable response. Long term implants are defined as items implanted for more than 30 days.

The present invention also provides a kit as defined in claim 15. The kit can comprise a saleable package comprising a first container that contains first monomer species (a), and a second container that contains second monomer species (b). Alternatively, the first and second monomer species can be contained in the same container. The kit can also comprise, if desired, a third container containing the polymerization initiator or accelerator.

The present invention also provides a use as defined in claim 7.

### DETAILED DESCRIPTION OF PREFERRED EMBODIMENTS

Adhesives of the present invention are biocompatible and may be applied internally or externally in or on living tissue.

The present invention employs at least one alkyl ester cyanoacrylate monomer having the formula 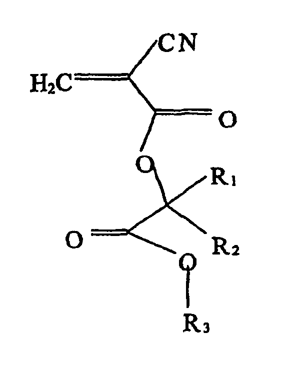 wherein R₁ and R₂ are independently H, a straight, branched or cyclic alkyl group, or are combined together in a cyclic alkyl group, and R₃ is a straight, branched or cyclic alkyl group.

The present invention also provides for the use with the monomers of quaternary amine polymerization initiators or accelerators such as quaternary amines having the formula 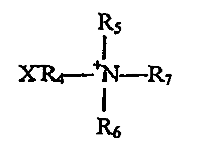 wherein R₄, R₅, R₆ and R₇ are each independently H or a substituted or unsubstituted straight, branched or cyclic alkyl group; a substituted or unsubstituted aromatic ring; a substituted or unsubstituted aralkyl group; or a substituted or unsubstituted alkyl or aromatic group which may include one or more hetero atom functionalities such as oxygen, sulfur, nitrogen, etc.; and X⁻ is an anion such as a halide, for example chloride, bromide, or fluoride, or hydroxyl; suitable quaternary amine initiators include but are not limited to domiphen bromide, butyrylcholine chloride, benzalkonium bromide and acetyl choline chloride.

Preferred monomer compositions of the present invention, and polymers formed therefrom, are useful as tissue adhesives, sealants for preventing bleeding or for covering open wounds, and in other biomedical applications. They find uses in, for example, preventing body fluid leakage, tissue approximation, apposing surgically incised or traumatically lacerated tissues; retarding blood flow from wounds; drug delivery; dressing burns; dressing skin or other superficial or deep tissue surface wounds (such as abrasions, chaffed or raw skin, and/or stomatitis); and aiding repair and regrowth of living tissue. Monomer compositions of the present invention, and polymers formed therefrom, have broad application for sealing wounds in various living tissue and internal organs, and can be applied, for example, on the interior or exterior of various organs or tissues. Monomer compositions of the present invention, and polymers formed therefrom, are also useful in industrial and home applications, for example in bonding rubbers, plastics, wood, composites, fabrics, and other natural and synthetic materials.

Monomers that may be used in this invention are readily polymerizable, e.g. anionically polymerizable or free radical polymerizable, or polymerizable by zwitterions or ion pairs to form polymers. Some such monomers are disclosed in, for example, U.S. Patent No. 5,328,687 to Leung, et al.

Alkyl ester cyanoacrylates can be prepared according to the procedure described in U.S. Patent No. 3,995,641 to Kronenthal et al. In the Kronenthal et al. method, such cyanoacrylate monomers are prepared by reacting an alkyl ester of an alpha-cyanoacrylic acid with a cyclic 1,3-diene to form a Diels-Alder adduct which is then subjected to alkaline hydrolysis followed by acidification to form the corresponding alpha-cyanoacrylic acid adduct. The alpha-cyanoacrylic acid adduct is preferably esterified by an alkyl bromoacetate to yield the corresponding carbalkoxymethyl alpha-cyanoacrylate adduct. Alternatively, the alpha-cyanoacrylic acid adduct may be converted to the alpha-cyanoacrylyl halide adduct by reaction with thionyl chloride. The alpha-cyanoacrylyl halide adduct is then reacted with an alkyl hydroxyacetate or a methyl substituted alkyl hydroxyacetate to yield the corresponding carbalkoxymethyl alpha-cyanoacrylate adduct or carbalkoxy alkyl alpha-cyanoacrylate adduct, respectively. The cyclic 1,3-diene blocking group is finally removed and the carbalkoxy methyl alpha-cyanoacrylate adduct or the carbalkoxy alkyl alpha-cyanoacrylate adduct is converted into the corresponding carbalkoxy alkyl alpha-cyanoacrylate by heating the adduct in the presence of a slight deficit of maleic anhydride.

Alkyl ester cyanoacrylates can also be prepared through the Knoevenagel reaction of an alkyl cyanoacetate, or an alkyl ester cyanoacetate, with paraformaldehyde. This leads to a cyanoacrylate oligomer. Subsequent thermal cracking of the oligomer results in the formation of a cyanoacrylate monomer. After further distillation, a cyanoacrylate monomer with high purity (greater than 95.0%, preferably greater than 99.0%, and more preferably greater than 99.8%), may be obtained.

Monomers prepared with low moisture content and essentially free of impurities (e.g., surgical grade) are preferred for biomedical use. Monomers utilized for industrial purposes need not be as pure.

Preferred alkyl ester cyanoacrylate monomers have the formula 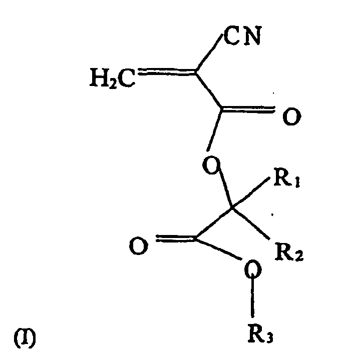 wherein R₁ and R₂ are, independently H, a straight, branched or cyclic alkyl, or are combined together in a cyclic alkyl group, and R₃ is a straight, branched or cyclic alkyl group. Preferably, R₁ is H or a C₁, C₂ or C₃ alkyl group, such as methyl or ethyl; R₂ is H or a C₁, C₂ or C₃ alkyl group, such as methyl or ethyl; and R₃ is a C₁-C₁₆ alkyl group, more preferably a C₁ -C₁₀ alkyl group, such as methyl, ethyl, propyl, isopropyl, butyl, isobutyl, pentyl, hexyl, heptyl, octyl, nonyl or decyl, and even more preferably a C₂, C₃ or C₄ alkyl group.

Examples of preferred alkyl ester cyanoacrylates include, but are not limited to, butyl lactoyl cyanoacrylate (BLCA), butyl glycoloyl cyanoacrylate (BGCA), isopropyl glycoloyl cyanoacrylate (IPGCA), ethyl lactoyl cyanoacrylate (ELCA), and ethyl glycoloyl cyanoacrylate (EGCA). BLCA may be represented by formula (I) above, wherein R₁ is H, R₂ is methyl and R₃ is butyl. BGCA may be represented by formula (I) above, wherein R₁ is H, R₂ is H and R₃ is butyl. IPGCA may be represented by formula (I) above, wherein R₁ is H, R₂ is H and R₃ is isopropyl. ELCA may be represented by formula (I) above, wherein R₁ is H, R₂ is methyl and R₃ is ethyl. EGCA may be represented by formula (I) above, wherein R₁ is H, R₂ is H and R₃ is ethyl. Other cyanoacrylates useful in the present invention are disclosed in U.S. Patent No. 3,995,641 to Kronenthal et al.

Alkyl ester cyanoacrylates are particularly useful for medical applications because of their absorbability by living tissue and associated fluids. According to the present invention, 100% of the polymerized and applied cyanoacrylate may be absorbed in a period of less than 2 years, such as approximately 2-24 months after application of the adhesive to living tissue. Alternatively, the absorption rate can be tailored to provide absorption rates of, for example, 3-18 months, including 3-6 months, 6-12 months, or 12-18 months. Of course, the present invention is not particularly limited to any absorption time, as the desired absorption time can vary depending on the particular uses and tissues involved. Thus, for example, longer absorption time may be desired where the adhesive composition is applied to hard tissues, such as bone, but a faster absorption time may be desired where the adhesive composition is applied to softer tissues.

The selection of monomer will affect the absorption rate of the resultant polymer, as well as the polymerization rate of the monomer. Two or more different monomers that have varied absorption and/or polymerization rates are used in combination to give a greater degree of control over the absorption rate of the resultant polymer, as well as the polymerization rate of the monomer. Thus, an important aspect of embodiments of the invention lies in the selection of the monomers and initiator to control within relatively narrow and predictable ranges both the polymerization and absorption rates.

According to the present invention, the adhesive composition comprises a mixture of monomer species. It is preferred that the absorption rates be sufficiently different that a mixture of the two monomers can yield a third absorption rate that is effectively different from the absorption rates of the two monomers individually. Thus, for example, it is preferred that the absorption rate of the faster absorbing monomer species be at least 10% faster than the absorption rate of the slower absorbing monomer species. More preferably, the absorption rate of the faster absorbing monomer species be at least 25% or 50% faster, or even 75% or 100% faster, than the absorption rate of the slower absorbing monomer species. The absorbable or faster absorbing/degrading monomer species is an alkyl ester cyanoacrylate, while the non-absorbable or slower absorbing/degrading monomer species is alkyl α-cyanoacrylate having an alkyl chain length of from 2 to 12, and preferably from 3 to 8 carbon atoms. Examples of such suitable monomers thus include, but are not limited to, octyl (such as 2-octyl), hexyl, and butyl α-cyanoacrylates.

In embodiments, the respective monomer species can be mixed in any suitable ratio to provide the desired degradation rate of the final polymer material. Thus, for example, suitable mixing ratios can range anywhere from about 1:99 or from about 10:90 to about 90:10 or about 99:1 in terms of parts by weight faster absorbing monomer to parts by weight non-absorbable or slower absorbing monomer. Preferred ratios are from about 15:85 to about 85:15 or from about 25:75 to about 75:25. For example, a desired degradation rate can be obtained by mixing faster absorbing monomer species and non-absorbable or slower absorbing monomer species in a weight ratio of about 50:50. In embodiments, a suitable composition can be obtained by mixing faster absorbing monomer species and non-absorbable or slower absorbing monomer species in a weight ratio of from about 40:60 to about 60:40. These ratios are particularly beneficial for achieving a desired balance between the relatively fast degradation rates of alkyl ester cyanoacrylates and the relatively slow degradation rates of other monomer species such as alkyl alpha-cyanoacrylates. However, these ratios and the present invention are in no way limited to such combinations.

For example, suitable compositions according to the present invention can be prepared by mixing suitable quantities of 2-octyl alpha-cyanoacrylate with one of butyl lactoyl cyanoacrylate (BLCA), butyl glycoloyl cyanoacrylate (BGCA), isopropyl glycoloyl cyanoacrylate (IPGCA), ethyl lactoyl cyanoacrylate (ELCA), and ethyl glycoloyl cyanoacrylate (EGCA). Preferably, such mixtures range from ratios of about 75:25 to about 25:75 by weight such as from about 60:40 to about 40:60 by weight.

In addition, although the above discussion is with respect to a composition containing only two different monomer species, the present invention is not limited to such an embodiment. Rather, as desired, the monomer composition can have two or more different monomer species, to provide further control over the absorption/degradation rate and other characteristics of the resultant polymer. Thus, for example, the composition can include two, three, four, five or even more different monomer species. Furthermore, where more than two monomer species are used, the various monomer species need not all have different absorption/degradation rates, although it is preferred that the monomer species individually provide at least two different absorption/degradation rates.

Some alkyl ester cyanoacrylate monomers may react slowly due to bulky alkyl groups, apparently limiting their applicability as surgical adhesives. By themselves, alkyl ester cyanoacrylates cure in several hours, or in some cases do not fully cure at all. To overcome problems associated with slow polymerization of the monomers, a compatible agent which initiates or accelerates polymerization of the alkyl ester cyanoacrylate monomer, may be used with the monomer composition. Initiators and accelerators particularly suitable for use with alkyl ester cyanoacrylates provide a fast cure rate while retaining the absorbable properties of the adhesive. Alkyl ester cyanoacrylates stimulated to cure by a suitable initiator or accelerator may be made to cure in as short as a few seconds to a few minutes. The cure rate may be closely controlled by selection of an amount or concentration of initiator or accelerator added to the cyanoacrylate and may thus be readily controlled by one skilled in the art in light of the present disclosure. A suitable initiator provides a consistent controllable complete polymerization of the monomer so that the polymerization of the monomer can be made to occur in the time desired for the particular application. Quaternary amine initiators or accelerators are particularly desirable with alkyl ester cyanoacrylate monomers for such reasons.

The initiator or accelerator may be in the form of a solid, such as a powder or a solid film, or in the form of a liquid, such as a viscous or paste-like material. The initiator or accelerator may also include a variety of additives, such as surfactants or emulsifiers. Preferably, the initiator or accelerator is soluble in the monomer composition, and/or comprises or is accompanied by at least one surfactant which, in embodiments, helps the initiator or accelerator co-elute with the monomer composition. In embodiments, the surfactant may help disperse the initiator or accelerator in the monomer composition.

The initiator or accelerator may be applied to tissue before the monomer composition, or may be applied directly to the monomer composition once the monomer composition is applied to tissue. In embodiments, the initiator or accelerator may be combined with the monomer composition just prior to applying the composition to tissue.

The selection of an initiator or accelerator may additionally affect the rate at which the polymerized monomer is absorbed by living tissue. Therefore, the most suitable initiators or accelerators are those that polymerize the monomer at a rate suitable for medical applications while providing a polymer that is substantially absorbed in less than two years. For the purposes of this invention, the phrase "suitable for medical application(s)" means that the initiator or accelerator polymerizes the monomer in less than 5 minutes or less than 3 minutes, preferably in less than 2.5 minutes, more preferably in less than 1 minute, and often in less than 45 seconds. Of course, the desired polymerization time can vary for different compositions and/or uses. Preferably, a suitable initiator or accelerator and a suitable monomer are selected to provide a polymer that is substantially absorbed by a living organism in 2-24 months, such as 3-18 months or 6-12 months after application of the adhesive to living tissue.

A suitable initiator or accelerator in a suitable quantity can be selected in light of the present disclosure, in combination with the selection of monomer, to produce a polymer with a desired absorption rate. A screening process utilizing routine experimentation may be used to identify combinations of monomers and initiators or accelerators that possess the desired reaction kinetics and produce a polymer that is absorbed *in vivo* in the desired period of time. Particularly beneficial initiators or accelerators, as well as monomers, are identified by the present disclosure. Therefore, for example, a butyl lactoyl cyanoacrylate monomer may be polymerized with, for example, domiphen bromide to test the polymerization rate. The quantity, or type, of initiator or accelerator or monomer may be adjusted if the desired polymerization rate is not achieved. Further, the polymer may be tested by *in vivo* application on animal (including human) tissue to determine absorption rates.

Depending, for example, on the necessary healing time for a wound, a corresponding absorption rate may be desired. Since healing times vary in different organisms and different tissues, the ability to control the absorption rate of the adhesive is beneficial to ensure that the adhesive polymer lasts long enough to provide time for the wound to heal, but absorbs within a reasonable time, preferably within 2 years from application of the adhesive to living tissue. Thus, according to the present invention, the absorption rate of the adhesive material can be selected in one of several ways. First, the absorption rate can be selected by determining desired specific monomer and initiator species. Thus, the absorption rate can be selected by proper selection of the desired monomer materials, and their relative mixing proportions, and optionally further by proper selection of a desired initiator or accelerator.

In preferred embodiments, the present invention provides for the use of quaternary amine polymerization initiators or accelerators such as quaternary amines having the formula 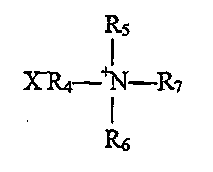 wherein R₄, R₅, R₆ and R₇ are each independently H or a substituted or unsubstituted straight, branched or cyclic alkyl group; a substituted or unsubstituted aromatic ring; a substituted or unsubstituted aralkyl group; or a substituted or unsubstituted alkyl or aromatic group which may include one or more hetero atom functionalities such as oxygen, sulfur, nitrogen, etc.; and X⁻ is an anion such as a halide, for example chloride, bromide, or fluoride, or hydroxyl. In preferred embodiments, at least one of R₄, R₅, R₆ and R₇ includes an aromatic group and/or a hetero atom functionality such as an ether or ester linkage or corresponding linkages where the hetero atom is sulfur or nitrogen. Preferred quaternary amine initiators are selected from the group consisting of domiphen bromide, butyrylcholine chloride, benzalkonium bromide and acetyl choline chloride.

Benzalkonium halides, such as benzalkonium chloride, are particularly preferred in embodiments. When used, the benzalkonium halide can be benzalkonium halide in its unpurified state, which comprises a mixture of varying chain-length compounds, or it can be any suitable purified compound including those having a chain length of from about 12 to about 18 carbon atoms, including but not limited to C₁₂, C₁₃, C₁₄, C₁₅, C₁₆, C₁₇, and C₁₈ compounds.

Domiphen bromide is preferred in other embodiments. Domiphen bromide may be represented by the following formula: 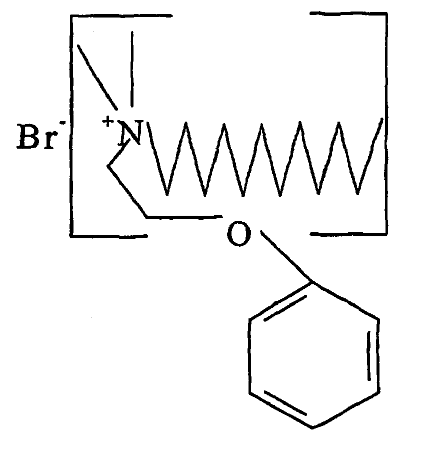 Butyrylcholine chloride may be represented by the following formula:

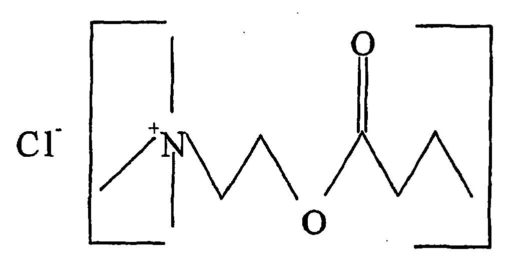

Initiators or accelerators, such as quaternary amines mentioned above, are preferably used in the present invention, but other initiators or accelerators may also be selected by one of ordinary skill in the art without undue experimentation. Such suitable initiators or accelerators may include, but are not limited to, detergent compositions; surfactants: e.g., nonionic surfactants such as polysorbate 20 (e.g., Tween 20^{TM} from ICI Americas), polysorbate 80 (e.g., Tween 80^{TM} from ICI Americas) and poloxamers, cationic surfactants such as tetrabutylammonium bromide, anionic surfactants such as sodium tetradecyl sulfate, and amphoteric or zwitterionic surfactants such as dodecyldimethyl(3-sulfopropyl)ammonium hydroxide, inner salt; amines, imines and amides, such as imidazole, tryptamine, urea, arginine and povidine; phosphines, phosphites and phosphonium salts, such as triphenylphosphine and triethyl phosphite; alcohols such as ethylene glycol, methyl gallate, ascorbic acid, tannins and tannic acid; inorganic bases and salts, such as sodium bisulfite, magnesium hydroxide, calcium sulfate and sodium silicate; sulfur compounds such as thiourea and polysulfides; polymeric cyclic ethers such as monensin, nonactin, crown ethers, calixarenes and polymeric epoxides; cyclic and acyclic carbonates, such as diethyl carbonate; phase transfer catalysts such as Aliquat 336; organometallics such as cobalt naphthenate and manganese acetylacetonate; and radical initiators or accelerators and radicals, such as di-t-butyl peroxide and azobisisobutyronitrile.

In embodiments, mixtures of two or more, such as three, four, or more, initiators or accelerators can be used. A combination of multiple initiators or accelerators may be beneficial, for example, to tailor the initiator of the polymerizable monomer species. For example, where a blend of monomers is used, a blend of initiators may provide superior results to a single initiator. For example, the blend of initiators or accelerators can provide one initiator that preferentially initiates one monomer, and a second initiator that preferentially initiates the other monomer, or can provide initiation rates to help ensure that both monomer species are initiated at equivalent, or desired non-equivalent, rates. In this manner, a blend of initiators can help minimize the amount of initiator necessary Furthermore, a blend of initiators may enhance the polymerization reaction kinetics.

Specific compositions of the invention may have various combinations of alkyl ester cyanoacrylates and thickeners, plasticizers, colorants, preservatives, heat dissipating agents, stabilizing agents and the like, which will be described in more detail below. Preferably, according to one embodiment of the present invention, a composition of this invention has from 65 to 99.9 weight % of monomer such as an alkyl ester cyanoacrylate or blend of cyanoacrylates and is promoted to polymerize by 0.005 to 10 weight % of an initiator or accelerator. More preferably, a composition of this invention has from 80 to 99.9 weight % of an alkyl ester cyanoacrylate and is promoted to polymerize by 0.02 to 5 weight % of an initiator or accelerator. Even more preferably, a composition of this invention has 85 to 99.9 weight % of monomer such as an alkyl ester cyanoacrylate, such as butyl lactoyl cyanoacrylate or a blend of cyanoacrylates, and is promoted to polymerize by 0.05 to 3 weight % of an initiator or accelerator, such as domiphen bromide.

Compositions of this invention may also include 0 to 25, more preferably 0 to 10, for example 0 to 5 weight % based on a total weight of the composition of at least one of the following: thickeners, plasticizers, colorants, preservatives, heat dissipating agents, stabilizing agents and the like. Of course, other compositions based on other proportions and/or components can readily be prepared according to embodiments of the present invention in light of the present disclosure.

Compositions of the present invention may be utilized in conjunction with other sealing means. For example, an adhesive may be applied to a wound that has been closed using surgical suture, tape, or staples. Adhesives of the present invention may also be used in conjunction with other sealing means, such as means identified in U.S. Patent No. 6,014,714.

Compositions of the present invention may be applied in single or multiple applications. The adhesives may be applied in a first layer, and after the first layer is allowed to fully or partially polymerize, a subsequent layer may be added. Such a process may be conducted numerous times, depending on the size of the wound and the amount of adhesive applied in each application.

The monomeric composition may be packaged in any type of suitable container fabricated from materials including, but not limited to, glass, plastic, metal packages, and film-formed packages. Suitable containers preferably include those into which the compositions may be dispensed and sterilized without unacceptable damage to, or degradation of, the container or the components of the monomer composition. Post-halogenated, such as fluorinated, polymeric barrier layers on at least the monomer-contacting surfaces of the container provide a superior shelf-life for monomer compositions, as disclosed in U.S. Patent Application No. 09/430,289, filed October 29, 1999. Glass is especially preferred when sterilization is achieved with dry heat because of the lack of stability of many plastics at temperatures used for dry heat sterilization. Examples of types of containers include, but are not limited to, ampoules, vials, syringes, pipettes, and the like.

The present invention also provides a saleable kit as defined in claim 15.

The kit can also include one or more other additives, including such additives as are described in detail below. When present, each of the additives can independently be either packaged separately from or in combination with the other additives or the monomer composition. As desired, the different monomer species can be packaged separately or together in suitable containers. If packaged separately, the kit provides the user the option of tailoring the absorption or degradation rate by suitably selecting a mixing ratio for the monomer species. The kit may comprise a second container containing the quaternary amine. Or, the first container could have the quaternary amine initiator or accelerator in or on it as long as the initiator or accelerator is not in contact with the monomer prior to the desired use.

The initiator or accelerator is selected so that it functions in conjunction with the co-packaged polymerizable monomer composition to initiate polymerization of at least one of, and preferably at least all of, the monomer species or to modify (e.g., accelerate) the rate of polymerization for the monomers to form a polymeric adhesive. The proper combination of initiator or accelerator and polymerizable monomer can be determined by one of ordinary skill in the art without undue experimentation in light of the present disclosure.

In each of the above embodiments, the kit may also include a suitable applicator, such as a brush, swab, sponge or the like, to assist in applying the composition to living tissue. If desired, the quaternary amine or other initiator and/or other additives can be located in or on the applicator.

The kit is also preferably sterilized; however, the containers and components may be sterilized separately or together. Preferably, kits and the kit components (including compositions) of the present invention have a sterility level in the range of 10^{- 3} to 10⁻⁶ Sterility Assurance Level (SAL) and are sterile for surgical purposes. Various designs of such kits are disclosed, for example, in U.S. Patent Application No. 09/385,030, filed August 30, 1999. The sterilization may be accomplished by techniques known to the skilled artisan, and is preferably accomplished by methods including, but not limited to, chemical, physical, and irradiation methods. Examples of physical methods include, but are not limited to, sterile fill, filtration, sterilization by heat (dry or moist) and retort canning. Examples of irradiation methods include, but are not limited to, gamma irradiation, electron beam irradiation, and microwave irradiation. Preferred methods are dry and moist heat sterilization and electron beam irradiation. In embodiments where a composition is to be used for medical applications, the sterilized composition should show low levels of toxicity to living tissue during its useable life.

In embodiments of the present invention, any suitable applicator may be used to apply the adhesive composition to a substrate. For example, the applicator may include an applicator body, which is formed generally in the shape of a tube having a closed end, an open end, and a hollow interior lumen, which holds a crushable or frangible ampoule. The applicator and its related packaging may be designed as a single-use applicator or as a multi-use applicator. Suitable multi-use applicators are disclosed, for example, in U.S. Patent Application No. 09/385,030, filed August 30, 1999.

In embodiments of the invention, the applicator may comprise elements other than an applicator body and an ampoule. For example, an applicator tip may be provided on the open end of the applicator. The applicator tip material may be porous, absorbent, or adsorbent in nature to enhance and facilitate application of the composition within the ampoule. Suitable designs for applicators and applicator tips that may be used according to the present invention are disclosed in, for example, U.S. Patent No. 5,928,611 to Leung and U.S. Patent Applications Nos. 09/069,979, filed April 30, 1998, 09/069,875, filed April 30, 1998, 09/479,059, filed January 7, 2000, and 09/479,060, filed January 7,2000.

In embodiments of the present invention, an applicator may contain the initiator or accelerator on a surface portion of the applicator or applicator tip, or on the entire surface of the applicator tip, including the interior and the exterior of the tip. When the initiator or accelerator is contained in or on an applicator tip, the initiator or accelerator may be applied to the surface of the applicator tip or may be impregnated or incorporated into the matrix or internal portions of the applicator tip. Additionally, the initiator or accelerator may be incorporated into the applicator tip, for example, during the fabrication of the tip.

In other embodiments, the initiator or accelerator may be coated on an interior surface of the applicator body and/or on an exterior surface of an ampoule or other container disposed within the applicator body, may be placed in the applicator body in the form of a second frangible vial or ampoule and/or may be otherwise contained within the applicator body, so long as a non-contacting relationship between the polymerizable monomer composition and the initiator or accelerator is maintained until use of the adhesive.

Various designs of applicators and methods for incorporating the initiator or accelerator into the applicator are disclosed in U.S. Patent No. 5,928,611 to Leung and U.S. Patent Applications Nos. 09/069,979, filed April 30, 1998, 09/069,875, filed April 30, 1998, 09/145,200, filed September 1, 1998, and 09/479,059 and 09/479,060, both filed January 7, 2000.

In embodiments, the polymerizable compositions according to the present invention can further comprise one or more suitable or desirable additives. When incorporated into the composition or used with the composition, it is preferred although not required that the additive or additives also be absorbable. Preferably, the additives have an absorption rate that is about comparable to the absorption rate of the resultant polymer material, although slower or faster absorption rates can be used, as desired.

The polymerizable compositions useful in the present invention may also further contain one or more preservatives, for prolonging the storage life of the composition. Suitable preservatives, and methods for selecting them and incorporating them into adhesive compositions, are disclosed in U.S. Patent Application No. 09/430,180, the entire disclosure of which is incorporated herein by reference.

Monomer compositions of the invention may also include a heat dissipating agent. Heat dissipating agents include liquids or solids that may be soluble or insoluble in the monomer. The liquids may be volatile and may evaporate during polymerization, thereby releasing heat from the composition. Suitable heat dissipating agents may be found in U.S. Patent No. 6,010,714 to Leung et al.

The composition or solution of the present invention may optionally include at least one plasticizing agent that assists in imparting flexibility to the polymer formed from the monomer. The plasticizing agent preferably contains little or no moisture and should not significantly affect the stability or polymerization of the monomer. Examples of suitable plasticizers include but are not limited to tributyl citrate, acetyl tri-n-butyl citrate (ATBC), polydimethylsiloxane, hexadimethylsilazane and others as listed in U.S. Patent Application Serial No. 09/471,392 filed December 23, 1999.

The composition or solution of the present invention may optionally also include thickeners. Suitable thickeners include those listed in U.S. Patent Application Serial No. 09/472,392 filed December 23,1999.

The composition or solution of the present invention may also optionally include at least one thixotropic agent. Examples of suitable thixotropic agents and thickeners are disclosed in, for example, U.S. Patent No. 4,720,513, and U.S. Patent Application Serial No. 09/374,207 filed August 12, 1999.

The composition or solution of the present invention may optionally also include one or more stabilizers, preferably both at least one anionic vapor phase stabilizer and at least one anionic liquid phase stabilizer. These stabilizing agents may inhibit premature polymerization. Suitable stabilizers may include those listed in U.S. Patent Application Serial No. 09/471,392 filed on December 23, 1999. Other stabilizing agents, such as free radical stabilizing agents, can also be included as desired.

Compositions or solutions of the present invention may also include at least one biocompatible agent effective to reduce active formaldehyde concentration levels produced during *in vivo* biodegradation of the polymer (also referred to herein as "formaldehyde concentration reducing agents"). Preferably, this component is a formaldehyde scavenger compound. Examples of formaldehyde scavenger compounds useful in this invention include sulfites; bisulfites; mixtures of sulfites and bisulfites, etc. Additional examples of formaldehyde scavenger compounds useful in this invention and methods for their implementation can be found in U.S. Patents Nos. 5,328,687, 5,514,371, 5,514,372, 5,575,997, 5,582,834 and 5,624,669, all to Leung et al.

The compositions of the present invention may also include pH modifiers to control the rate of degradation of the resulting polymer, as disclosed in U.S. Patent Application No. 08/714,288, filed September 18, 1996.

To improve the cohesive strength of the compositions or solutions of this invention, difunctional monomeric cross-linking agents may be added to monomer compositions of this invention. Such crosslinking agents are known. U.S. Patent No. 3,940,362 to Overhults discloses exemplary cross-linking agents.

The compositions or solutions of this invention may further contain fibrous reinforcement and colorants such as dyes, pigments, and pigment dyes. Examples of suitable fibrous reinforcement include PGA microfibrils, collagen microfibrils, and others as described in U.S. Patent Application Serial No. 09/471,392 filed on December 23, 1999.

Other modifications to compositions of the present invention are exemplified by U.S. Patents Nos. 5,624,669; 5,582,834; 5,575,997; 5,514,371; 5,514,372; and 5,259,835; and U.S. Patent Application No. 08/714,288.

Although not limited to any particular formulation, a particular composition suitable for use in the present invention comprises a blend of butyl lactoyl cyanoacrylate (BLCA) and octyl cyanoacrylate (OCA). Suitable blends preferably range from about 25:75 to about 40:60 (weight ratio BLCA:OCA). The composition also preferably includes a suitable stabilizer system, such as one comprising specified amounts of sulfuric acid (such as about 25 to about 100 ppm of sulfuric acid, preferably about 20 ppm), sulfur dioxide (such as about 1 to about 50 ppm, preferably about 10 to about 12 ppm), hydroquinone (such as about 100 to about 2000 ppm, preferably about 960 to about 1200 ppm), p-methoxyphenol (such as about 10 to about 200 ppm, preferably about 96 to about 120 ppm), and butylated hydroxyanisole (such as about 100 to about 10,000 ppm, preferably about 500 to about 800 ppm). The composition can include additional materials, such as a colorant such as D & C violet #2 (such as 20 to about 2000 ppm, preferably about 35 to 100 ppm) and the like. Suitable initiators can include, for example, domiphen bromide or benzalkonium chloride, in amounts ranging from about 100 to about 15,000 ppm.

### EXAMPLES

The present invention will be further understood by reference to the following non-limiting examples.

### Reference Example 1:

70 µl of butyl lactoyl cyanoacrylate are mixed with 2.5 µmoles of domiphen bromide as the monomer is passed through a porous applicator tip. The resulting mixture sets in approximately 40 seconds. In these Examples, "setting time" is measured as the time when the material reaches its maximum exotherm.

### Reference Example 2:

36 µl of butyl lactoyl cyanoacrylate are mixed with 0.625 µmoles of butyrylcholine chloride as the monomer is passed through a porous applicator tip. The resulting mixture sets in approximately 60 seconds.

### Reference Example 3:

A polymer is formed from the initiation of butyl lactoyl cyanoacrylate monomer with domiphen bromide in situ on a polypropylene mesh and placed in a phosphate buffer at 39°C. Samples are rinsed, dried and weighed, and the degradation results of the polymer are shown in the table below, wherein Mn is the number average molecular weight of the sample.

| | | | | | | |
|---|---|---|---|---|---|---|
| Time (days) | 0 | 28 | 56 | 84 | 112 | 140 |
| Mass loss (%) | 0 | 8 | 18 | 31 | 45 | 60 |
| Mn x 1,000 | 84 | 12 | 6.2 | 3.1 | 1.9 | 1.3 |

A polymer is formed from the initiation of butyl lactoyl cyanoacrylate monomer with azobisisobutyronitrile in situ on a polypropylene mesh and placed in a phosphate buffer at 39°C. Samples are rinsed, dried and weighed, and the degradation results of the polymer are shown in the table below, wherein Mn is the number average molecular weight of the sample.

| | | | | | | |
|---|---|---|---|---|---|---|
| Time (days) | 0 | 28 | 56 | 98 | 112 | 140 |
| Mass loss (%) | 0 | 3 | 6 | 9 | 10 | 12 |
| Mn x 1,000 | 28 | 23 | 23 | 21 | ― | 20 |

### Reference Example 4:

An absorbable adhesive polymer is formulated by the combination of:

| | |
|---|---|
| Butyl lactoyl cyanoacrylate monomer | 98.2600% (by weight); |
| Domiphen bromide | 1.7300% (by weight); |
| H₂SO₄ | 0.0025% (by weight); and |
| Butylated hydroxyanisole | 0.0075% (by weight). |

### Example 5:

Various monomeric adhesive compositions are formulated using varying amounts of butyl lactoyl cyanoacrylate (BLCA) and 2-octyl alpha-cyanoacrylate (2OCA). The compositions as formulated also include about 20 ppm sulfuric acid, 0 to 20 ppm sulfur dioxide, 0 to 2000 ppm hydroquinone, 0 to 180 ppm p-methoxyphenol, and 0 to 2000 ppm butylated hydroxyanisole. The mixing ratios of the monomers are set forth in the following Table. The compositions are initiated with domiphen bromide and applied to a surface, and the setting time of the compositions are measured. The setting time results are also set forth in the following Table.

| Sample | wt% BLCA | wt%2OCA | Setting Time (s) |
|---|---|---|---|
| A | 0 | 100 | 92 |
| B | 25 | 75 | 60 |
| C | 50 | 50 | 49 |
| D | 75 | 25 | 45 |
| E | 100 | 0 | 42 |

### Example 6:

The same compositions as used in Example 5 are *tested in vitro* for their absorption/degradation rates. As in Example 5, the compositions are formulated using varying amounts of butyl lactoyl cyanoacrylate (BLCA) and 2-octyl alpha-cyanoacrylate (2OCA). The mixing ratios of the monomers are set forth in the following Table.

Samples for *in vitro* degradation testing are prepared by initiating a quantity of the respective monomer composition and expressing it onto a pre-weighed polypropylene mesh having a thickness of approximately 0.19 mm and cut to dimensions of approximately 10 mm x 35 mm. The mesh is sandwiched between two surfaces of ultra high molecular weight polyethylene, which are separated by 0.203 mm thick stainless steel shims. After curing, the samples are removed from the mold and excess polymerized material is trimmed away. A portion of the trimmed away material is used for determining the starting molecular weight of each sample.

The samples are placed into sterilized extraction thimbles to minimize contact with the polymer material. The samples are then placed in sterile glass vials and filled with 21 ml of Dulbecco's phosphate buffered saline (PBS) with antibiotic/antimycotin added. The vials are placed in a water bath at 39°C. The PBS solution is exchanged weekly.

At intervals of 7 and 13 days post-polymerization, the formed polymer is tested to determine the absorption/degradation of the polymer. The absorption/degradation is measured by determining the change in mass% of the formed polymer. The testing is conducted by removing the sample from the buffer solution and rinsing them three times with sterile water. The samples are dried for 24 hours in vacuo before re-weighing. The measurements are also set forth in the following Table.

| | | | Mass% Change | |
|---|---|---|---|---|
| Sample | wt% BLCA | wt% 2OCA | 7 days | 13 days |
| A | 0 | 100 | -0.4 | -0.9 |
| B | 25 | 75 | -1.4 | -2.1 |
| C | 50 | 50 | -2.5 | -3.4 |
| D | 75 | 25 | -3.6 | -5.4 |
| E | 100 | 0 | -4.8 | -7.8 |

## Claims

1. A biocompatible adhesive composition, comprising: a first monomer species; and a second monomer species different from said first monomer species, wherein at least said first monomer species is absorbable, and an absorption rate of said first monomer species is different from an absorption rate of said second monomer species, wherein:
said first monomer species is an alkyl ester cyanoacrylate of formula 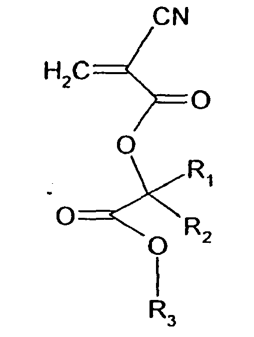 wherein R₁ and R₂ are independently H, a straight, branched or cyclic alkyl group, or are combined together in a cyclic alkyl group, and R₃ is a straight, branched or cyclic alkyl group; and
said second monomer species is an alkyl α-cyanoacrylate, having an alkyl group of from 2 to 12 carbon atoms.

2. The biocompatible adhesive composition of claim 1, wherein said alkyl ester cyanoacrylate is selected from the group consisting of butyl lactoyl cyanoacrylate, butyl glycoloyl cyanoacrylate, isopropyl glycoloyl cyanoacrylate, ethyl lactoyl cyanoacrylate, and ethyl glycoloyl cyanoacrylate.

3. The biocompatible adhesive composition of claim 1, wherein a weight ratio of said first monomer species to said second monomer species is from about 25:75 to about 75:25.

4. The biocompatible adhesive composition of claim 1, wherein said composition further comprises at least one additive selected from the group consisting of anionic stabilizing agents, free radical stabilizing agents, colorants, and plasticizers.

5. The biocompatible adhesive composition of claim 1, wherein said composition comprises:
a monomer blend comprising from about 25 to about 40 parts by weight butyl lactoyl cyanoacrylate and from about 60 to about 75 parts by weight octyl cyanoacrylate(OCA) ;
at least one anionic stabilizer ; and
at least one radical stabilizer.

6. The biocompatible adhesive composition of claim 5, wherein said at least one anionic stabilizer comprises about 25 to about 100 ppm of sulfuric acid and from about 1 to about 50 ppm sulfur dioxide, and said at least one radical stabilizer comprises from about 100 to about 2000 ppm hydroquinone, from about 10 to about 200 ppm p-methoxyphenol, and from about 100 to about 10,000 ppm butylated hydroxyanisole.

7. Use of an alkyl ester cyanoacrylate of formula 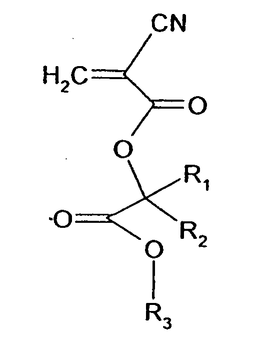 wherein R₁ and R₂ are independently H, a straight, branched or cyclic alkyl group, or are combined together in a cyclic alkyl group, and R₃ is a straight, branched or cyclic alkyl group;
an alkyl α-cyanoacrylate, having an alkyl group of from 2 to 12 carbon atoms; and
a polymerization initiator or accelerator, wherein said polymerization initiator or accelerator is a quaternary amine,
for the manufacture of a biocompatible adhesive for treating living tissue.

8. The use of claim 7, wherein the living tissue is internal tissue.

9. The use of claim 7, wherein two or more polymerization initiators or accelerators are applied to said living tissue.

10. The use of claim 7, wherein the polymerization initiator or accelerator is combined with at least one monomer prior to applying the composition to living tissue.

11. The use of claim 7, wherein the polymerization initiator or accelerator has the following formula:
X⁻R₄N⁺R₅R₆R₇
wherein R₄,R₅, R₆ and R₇ are each independently H or a substituted or unsubstituted straight, branched or cyclic alkyl group; a substituted or unsubstituted aromatic group; a substituted or unsubstituted aralkyl group; or a substituted or unsubstituted alkyl or aromatic group containing one or more hetero atoms; and X' is an anion.

12. The use of claim 11, wherein at least one of R₄, R₅, R₆ and R₇ comprises at least one member selected from the group consisting of an aromatic ring and a hetero atom-containing linkage.

13. The use of claim 11, wherein X⁻ is a halogen radical.

14. The use of claim 7, wherein said polymerization initiator or accelerator is at least one member selected from the group consisting of domiphen bromide, butyrylcholine chloride, benzalkonium bromide, and acetyl choline chloride.

15. A kit comprising a saleable package comprising:
(a) a first container that contains abiocompatible adhesive composition comprising: an alkyl ester cyanoacrylate of formula 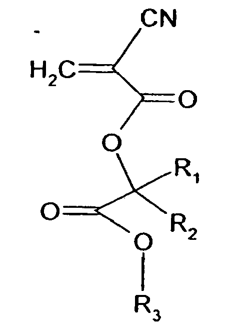 wherein R₁ and R₂ are independently H, a straight, branched or cyclic alkyl group, or are combined together in a cyclic alkyl group, and R₃ is a straight, branched or cyclic alkyl group;
(b) an alkyl α-cyanoacrylate, having an alkyl group of from 2 to 12 carbon atoms; and
(c) a polymerization initiator or accelerator, wherein said polymerization initiator or accelerator is a quaternary amine.

16. The kit of claim 15, wherein said polymerization initiator or accelerator is contained in a separate container not in contact with said biocompatible adhesive composition.

17. The kit of claim 15, wherein said polymerization initiator or accelerator is a quaternary amine having the following formula:
X⁻R₄N⁺R₅R₆R₇
wherein R₄,R₅, R₆ and R₇ are each independently H or a substituted or unsubstituted straight, branched or cyclic alkyl group; a substituted or unsubstituted aromatic group; a substituted or unsubstituted aralkyl group; or a substituted or unsubstituted alkyl or aromatic group containing one or more hetero atoms; and X⁻ is an anion.

18. The kit of claim 17, wherein at least one of R₄, R₅, R₆ and R₇ comprises at least one member selected from the group consisting of an aromatic ring and a hetero atom-containing linkage.

19. The kit of claim 17, wherein X⁻ is a halogen radical.

20. The kit of claim 15, wherein said polymerization initiator or accelerator is at least one member selected from the group consisting of domiphen bromide, butyrylcholine chloride, benzalkonium bromide, and acetyl choline chloride.

21. A polymerized film formed from the polymerization of a biocompatible adhesive composition according to claim 1.

## Patentansprüche

1. Biokompatible Klebstoffzusammensetzung, umfassend: eine erste Monomerart; und eine zweite Monomerart, die unterschiedlich zu der ersten Monomerart ist, wobei zumindest die erste Monomerart absorbierbar ist und eine Absorptionsrate der ersten Monomerart unterschiedlich zu einer Absorptionsrate der zweiten Monomerart ist, wobei:
die erste Monomerart ein Alkylestercyanoacrylat von Formel 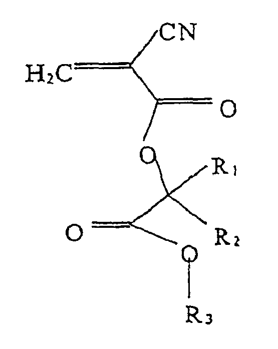 ist, in der R₁ und R₂ unabhängig voneinander H, eine geradkettige, verzweigte oder cyclische Alkylgruppe sind oder untereinander zu einer cyclischen Alkylgruppe verbunden sind, und R₃ eine geradkettige, verzweigte oder cyclische Alkylgruppe ist; und
die zweite Monomerart ein Alkyl-α-cyanoacrylat mit einer Alkylgruppe mit von 2 bis 12 Kohlenstoffatomen ist.

2. Biokompatible Klebstoffzusammensetzung nach Anspruch 1, wobei das Alkylestercyanoacrylat ausgewählt ist aus der Gruppe, bestehend aus Butyllactoylcyanoacrylat, Butylglykoloylcyanoacrylat, Isopropylglykoloylcyanoacrylat, Ethyllactoylcyanoacrylat und Ethylglykoloylcyanoacrylat.

3. Biokompatible Klebstoffzusammensetzung nach Anspruch 1, wobei das Gewichtsverhältnis der ersten Monomerart zur zweiten Monomerart von etwa 25:75 bis etwa 75:25 reicht.

4. Biokompatible Klebstoffzusammensetzung nach Anspruch 1, wobei die Zusammensetzung ferner zumindest einen Zusatzstoff umfasst, ausgewählt aus der Gruppe, bestehend aus anionischen Stabilisierern, Stabilisierern für freie Radikale, Färbemitteln und Weichmachern.

5. Biokompatible Klebstoffzusammensetzung nach Anspruch 1, wobei die Zusammensetzung:
eine Monomermischung, die von etwa 25 bis etwa 40 Gewichtsteile Butyllactoylcyanoacrylat und von etwa 60 bis etwa 75 Gewichtsteile Octylcyanoacrylat (OCA) umfasst;
zumindest einen anionischen Stabilisierer; und
zumindest einen Stabilisierer für Radikale
umfasst.

6. Biokompatible Klebstoffzusammensetzung nach Anspruch 5, wobei zumindest ein anionischer Stabilisierer etwa 25 bis etwa 100 ppm Schwefelsäure und von etwa 1 bis etwa 50 ppm Schwefeldioxid umfasst und zumindest ein Stabilisierer für Radikale von etwa 100 bis etwa 2.000 ppm Hydrochinon, von etwa 10 bis etwa 200 ppm p-Methoxyphenol und von etwa 100 bis etwa 10.000 ppm butyliertes Hydroxyanisol umfasst.

7. Verwendung eines Alkylestercyanoacrylats von Formel 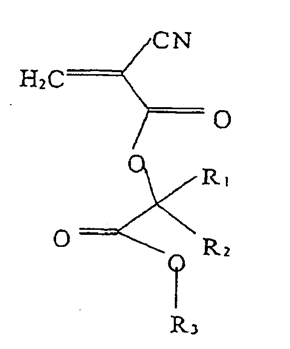 in der R₁ und R₂ unabhängig voneinander H, eine geradkettige, verzweigte oder cyclische Alkylgruppe sind, oder untereinander zu einer cyclischen Alkylgruppe verbunden sind, und R₃ eine geradkettige, verzweigte oder cyclische Alkylgruppe ist;
eines Alkyl-α-cyanoacrylats mit einer Alkylgruppe mit von 2 bis 12 Kohlenstoffatomen; und
eines Polymerisationsinitiators oder -beschleunigers, wobei der Polymerisationsinitiator oder -beschleuniger ein quartäres Amin ist,
zur Herstellung eines biokompatiblen Klebstoffes zur Behandlung von lebendem Gewebe.

8. Verwendung nach Anspruch 7, wobei das lebende Gewebe ein inneres Gewebe ist.

9. Verwendung nach Anspruch 7, wobei zwei oder mehr Polymerisationsinitiatoren oder -beschleuniger auf das lebende Gewebe aufgebracht werden.

10. Verwendung nach Anspruch 7, wobei der Polymerisationsinitiator oder -beschleuniger mit zumindest einem Monomer zusammengebracht wird, bevor die Zusammensetzung auf lebendes Gewebe aufgebracht wird.

11. Verwendung nach Anspruch 7, wobei der Polymerisationsinitiator oder -beschleuniger die folgende Formel aufweist:
X⁻R₄N⁺R₅R₆R₇
in der R₄, R₅, R₆ und R₇ jeweils unabhängig voneinander H oder eine substituierte oder unsubstituierte geradkettige, verzweigte oder cyclische Alkylgruppe; eine substituierte oder unsubstituierte aromatische Gruppe; eine substituierte oder unsubstituierte Aralkylgruppe; oder eine substituierte oder unsubstituierte Alkyl- oder aromatische Gruppe, die ein oder mehrere Heteroatome enthält, sind; und X⁻ ein Anion ist.

12. Verwendung nach Anspruch 11, wobei zumindest einer von R₄, R₅, R₆ und R₇ zumindest ein Mitglied umfasst, das ausgewählt ist aus der Gruppe, bestehend aus einem aromatischen Ring und einer ein Heteroatom enthaltenden Verknüpfung.

13. Verwendung nach Anspruch 11, wobei X⁻ ein Halogenrest ist.

14. Verwendung nach Anspruch 7, wobei der Polymerisationsinitiator oder -beschleuniger zumindest ein Mitglied ist, das ausgewählt ist aus der Gruppe, bestehend aus Domiphenbromid, Butyrylcholinchlorid, Benzalkoniumbromid und Acetylcholinchlorid.

15. Kit, umfassend eine verkaufsfertige Packung, umfassend:
(a) einen ersten Behälter, der eine biokompatible Klebstoffzusammensetzung enthält, umfassend: ein Alkylestercyanoacrylat von Formel 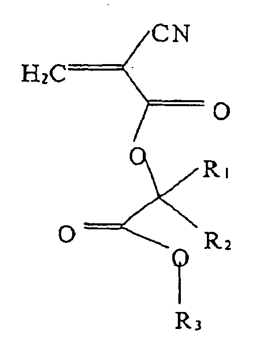 in der R₁ und R₂ unabhängig voneinander H, eine geradkettige, verzweigte oder cyclische Alkylgruppe sind, oder untereinander zu einer cyclischen Alkylgruppe verbunden sind, und R₃ eine geradkettige, verzweigte oder cyclische Alkylgruppe ist;
(b) ein Alkyl-α-cyanoacrylat mit einer Alkylgruppe mit 2 von 12 Kohlenstoffatomen; und
(c) einen Polymerisationsinitiator oder -beschleuniger, wobei der Polymerisationsinitiator oder -beschleuniger ein quartäres Amin ist.

16. Kit nach Anspruch 15, wobei der Polymerisationsinitiator oder -beschleuniger in einem getrennten Behälter enthalten ist, der nicht in Kontakt mit der biokompatiblen Klebstoffzusammensetzung steht.

17. Kit nach Anspruch 15, wobei der Polymerisationsinitiator oder -beschleuniger ein quartäres Amin mit der folgenden Formel ist:
X⁻R₄N⁺R₅R₆R₇
in der R₄, R₅, R₆ und R₇ jeweils unabhängig voneinander H oder eine substituierte oder unsubstituierte geradkettige, verzweigte oder cyclische Alkylgruppe; eine substituierte oder unsubstituierte aromatische Gruppe; eine substituierte oder unsubstituierte Aralkylgruppe; oder eine substituierte oder unsubstituierte Alkyl- oder aromatische Gruppe, die ein oder mehrere Heteroatome enthält, sind; und X⁻ ein Anion ist.

18. Kit nach Anspruch 17, wobei zumindest einer von R₄, R₅, R₆ und R₇ zumindest ein Mitglied umfasst, das ausgewählt ist aus der Gruppe, bestehend aus einem aromatischen Ring und einer ein Heteroatom enthaltenden Verknüpfung.

19. Kit nach Anspruch 17, wobei X⁻ ein Halogenrest ist.

20. Kit nach Anspruch 15, wobei der Polymerisationsinitiator oder -beschleuniger wenigstens ein Mitglied ist, das ausgewählt ist aus der Gruppe, bestehend aus Domiphenbromid, Butyrylcholinchlorid, Benzalkoniumbromid und Acetylcholinchlorid.

21. Polymerisierter Film, gebildet aus der Polymerisation einer biokompatiblen Klebstoffzusammensetzung nach Anspruch 1.

## Revendications

1. Composition d'adhésif biocompatible, comprenant : une première espèce monomère ; et une deuxième espèce monomère différente de ladite première espèce monomère, dans laquelle au moins ladite première espèce monomère est absorbable, et une vitesse d'absorption de ladite première espèce monomère est différente d'une vitesse d'absorption de ladite deuxième espèce monomère, dans laquelle ladite première espèce monomère est un cyanoacrylate d'ester alkylique de formule : 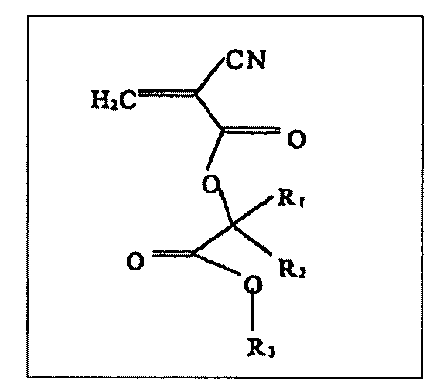 dans laquelle R₁ et R₂ sont indépendamment H, un groupe alkyle à chaîne droite, ramifiée ou cyclique ou bien sont combinés ensemble en un groupe alkyle cyclique, et R₃ est un groupe alkyle à chaîne droite, ramifiée ou cyclique, et
ladite deuxième espèce monomère est un α-cyanoacrylate d'alkyle ayant un groupe alkyle comptant de 2 à 12 atomes de carbone.

2. Composition d'adhésif biocompatible selon la revendication 1, dans laquelle ledit cyanoacrylate d'ester alkylique est choisi dans le groupe constitué par le cyanoacrylate de butyllactoyle, le cyanoacrylate de butylglycoloyle, le cyanoacrylate d'isopropylglycoloyle, le cyanoacrylate d'éthyllactoyle et le cyanoacrylate d'éthylglycoloyle.

3. Composition d'adhésif biocompatible selon la revendication 1, dans laquelle un ratio en poids de ladite première espèce monomère sur ladite deuxième espèce monomère se situe dans la plage d'environ 25:75 à environ 75:25.

4. Composition d'adhésif biocompatible selon la revendication 1, dans laquelle ladite composition comprend, en outre, un additif choisi dans le groupe constitué par des agents stabilisateurs anioniques, des agents stabilisant les radicaux libres, des colorants et des plastifiants.

5. Composition d'adhésif biocompatible selon la revendication 1, dans laquelle ladite composition comprend :
un mélange de monomères comprenant environ 25 à environ 40 parties en poids de cyanoacrylate de butyllactoyle et environ 60 à environ 75 parties en poids de cyanoacrylate d'octyle (OCA) ;
au moins un stabilisateur anionique ; et
au moins un stabilisateur de radicaux.

6. Composition d'adhésif biocompatible selon la revendication 5, dans laquelle ledit au moins un stabilisateur anionique comprend environ 25 à environ 100 ppm d'acide sulfurique et environ 1 à environ 50 ppm de dioxyde de soufre, et ledit au moins un stabilisateur de radicaux comprend environ 100 à environ 2000 ppm d'hydroquinone, environ 10 à environ 200 ppm de p-méthoxyphénol, et environ 100 à environ 10 000 ppm d'hydroxyanisole butylé.

7. Utilisation d'un cyanoacrylate d'ester alkylique de formule : 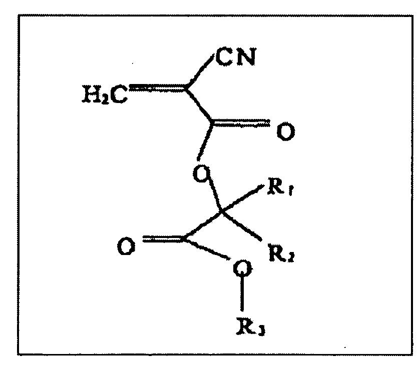 dans laquelle R₁ et R₂ sont indépendamment H, un groupe alkyle à chaïne droite, ramifiée ou cyclique ou bien sont combinés ensemble en un groupe alkyle cyclique, et R₃ est un groupe alkyle à chaîne droite, ramifiée ou cyclique ;
un α-cyanoacrylate d'alkyle ayant un groupe alkyle comptant de 2 à 12 atomes de carbone ; et
un amorceur ou accélérateur de polymérisation, ledit amorceur ou accélérateur de polymérisation étant une amine quaternaire pour la fabrication d'un adhésif biocompatible pour le traitement de tissu vivant.

8. Utilisation selon la revendication 7, dans laquelle le tissu vivant est un tissu interne.

9. Utilisation selon la revendication 7, dans laquelle deux ou plusieurs amorceurs ou accélérateurs de polymérisation sont appliqués sur ledit tissu vivant.

10. Utilisation selon la revendication 7, dans laquelle l'amorceur ou accélérateur de polymérisation est combiné avec le au moins un monomère avant l'application de la composition sur le tissu vivant.

11. Utilisation selon la revendication 7, dans laquelle ledit amorceur ou accélérateur de polymérisation a la formule suivante :
X⁻R₄N⁺R₅R₆R₇
dans laquelle R₄, R₅, R₆ et R₇ sont chacun indépendamment H ou un groupe alkyle à chaîne droite, ramifiée ou cyclique, substitué ou non substitué; un groupe aromatique substitué ou non substitué ; un groupe aralkyle substitué ou non substitué ; ou un groupe alkyle ou aromatique substitué ou non substitué contenant un ou plusieurs hétéroatomes ; et X⁻ est un anion.

12. Utilisation selon la revendication 11, dans laquelle l'un au moins parmi R₄, R₅, R₆ et R₇ comprend au moins un élément choisi dans le groupe constitué par un noyau aromatique et une liaison contenant un hétéroatome.

13. Utilisation selon la revendication 11, dans laquelle X⁻ est un radical halogène.

14. Utilisation selon la revendication 7, dans laquelle ledit amorceur ou accélérateur de polymérisation est au moins un élément choisi dans le groupe constitué par le bromure de domiphène, le chlorure de butyrylcholine, le bromure de benzalkonium et le chlorure d'acétylcholine.

15. Kit comprenant un emballage commercialisable comprenant :
a) un premier récipient qui contient une composition d'adhésif biocompatible comprenant un cyanoacrylate d'ester alkylique de formule 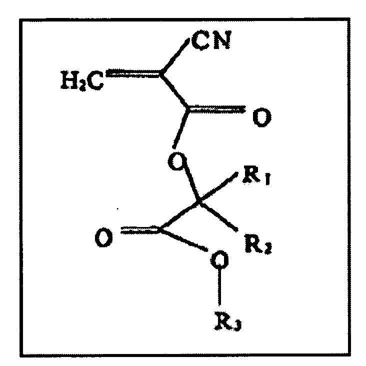 dans lequel
R₁ et R₂ sont indépendamment H, un groupe alkyle à chaîne droite, ramifiée ou cyclique ou bien sont combinés ensemble en un groupe alkyle cyclique, et R₃ est un groupe alkyle à chaîne droite, ramifiée ou cyclique ; et
b) un α-cyanoacrylate d'alkyle ayant un groupe alkyle comptant de 2 à 12 atomes de carbone ;
c) un amorceur ou accélérateur de polymérisation, ledit amorceur ou accélérateur de polymérisation étant une amine quaternaire.

16. Kit selon la revendication 15, dans lequel l'amorceur ou l'accélérateur de polymérisation est contenu dans un récipient séparé qui n'est pas en contact avec la composition d'adhésif biocompatible.

17. Kit selon la revendication 15, dans lequel l'amorceur ou l'accélérateur de polymérisation est une amine quaternaire ayant la formule suivante :
X⁻R₄N⁺R₅R₆R₇
dans laquelle R₄, R₅, R₆ et R₇ sont chacun indépendamment H ou un groupe alkyle à chaîne droite, ramifiée ou cyclique, substitué ou non substitué; un groupe aromatique substitué ou non substitué ; un groupe aralkyle substitué ou non substitué ; ou un groupe alkyle ou aromatique substitué ou non substitué contenant un ou plusieurs hétéroatomes ; et X⁻ est un anion.

18. Kit selon la revendication 17, dans lequel l'un au moins parmi R₄, R₅, R₆ et R₇ comprend au moins un élément choisi dans le groupe constitué par un noyau aromatique et une liaison contenant un hétéroatome.

19. Kit selon la revendication 17, dans lequel X⁻ est un radical halogène.

20. Kit selon la revendication 15, dans lequel ledit amorceur ou accélérateur de polymérisation est au moins un élément choisi dans le groupe constitué par le bromure de domiphène, le chlorure de butyrylcholine, le bromure de benzalkonium et le chlorure d'acétylcholine.

21. Film polymérisé formé par polymérisation d'une composition d'adhésif biocompatible selon la revendication 1.
